# EUROPEAN PATENT SPECIFICATION

(11) **EP 3 134 513 B1**
(45) Date of publication and mention of the grant of the patent: **06.03.2019**
(21) Application number: 15721045.1
(22) Date of filing: 20.04.2015
(51) Int. Cl.: C12N 5/074, C12N 5/071

(54) **METHOD OF PRODUCING SKIN-DERIVED PRECURSOR CELLS**
VERFAHREN ZUR HERSTELLUNG VON HAUTABGELEITETEN VORLÄUFERZELLEN
PROCÉDÉ DE PRODUCTION DE CELLULES PRÉCURSEURS D'ORIGINE CUTANÉE

(30) Priority: 21.04.2014 JP 2014087247; 24.12.2014 JP 2014260434
(43) Date of publication of application: 01.03.2017
(73) Proprietor: Kao Corporation, Tokyo 103-8210 (JP)
(72) Inventor: NAKAGIRI, Yoriko, Haga-gun Tochigi 321-3497 (JP)
(74) Representative: Vossius & Partner Patentanwälte Rechtsanwälte mbB
(86) International application number: PCT/JP2015/002156
(87) International publication number: WO 2015/162908

(56) References cited:
- WO-A1-2005/071063
- WO-A2-2007/016485
- WO-A2-2010/011352
- TOMA J G ET AL: "Isolation and characterization of multipotent skin-derived precursors from human skin", STEM CELLS, ALPHAMED PRESS, DAYTON, OH, US, vol. 23, 1 July 2005 (2005-07-01), pages 727-737, XP003011062, ISSN: 1066-5099
- JEFFREY BIERNASKIE ET AL: "SKPs Derive from Hair Follicle Precursors and Exhibit Properties of Adult Dermal Stem Cells", CELL STEM CELL, vol. 5, no. 6, 1 December 2009 (2009-12-01), pages 610-623, XP055198752, ISSN: 1934-5909, DOI: 10.1016/j.stem.2009.10.019
- REBECCA P. HILL ET AL: "Generation and Characterization of Multipotent Stem Cells from Established Dermal Cultures", PLOS ONE, vol. 7, no. 11, 30 November 2012 (2012-11-30), page e50742, XP055198753, DOI: 10.1371/journal.pone.0050742
- HUNT D P J ET AL: "Multipotent skin-derived precursors: from biology to clinical translation", CURRENT OPINION IN BIOTECHNOLOGY, LONDON, GB, vol. 20, no. 5, 1 October 2009 (2009-10-01), pages 522-530, XP026785518, ISSN: 0958-1669, DOI: 10.1016/J.COPBIO.2009.10.004 [retrieved on 2009-11-05]
- L. ALONSO ET AL: "Stem cells in the skin: waste not, Wnt not", GENES & DEVELOPMENT, vol. 17, no. 10, 15 May 2003 (2003-05-15), pages 1189-1200, XP055200245, ISSN: 0890-9369, DOI: 10.1101/gad.1086903
- XINYUE WANG ET AL: "Three-dimensional hydrogel scaffolds facilitate in vitro self-renewal of human skin-derived precursors", ACTA BIOMATERIALIA, vol. 10, no. 7, 28 March 2014 (2014-03-28) , pages 3177-3187, XP055200306, ISSN: 1742-7061, DOI: 10.1016/j.actbio.2014.03.018
- KAZUTOSHI TAKAHASHI ET AL: "Induction of Pluripotent Stem Cells from Adult Human Fibroblasts by Defined Factors", CELL, CELL PRESS, US, vol. 131, no. 5, 30 November 2007 (2007-11-30), pages 861-872, XP008155962, ISSN: 0092-8674, DOI: 10.1016/J.CELL.2007.11.019 cited in the application
- Ruifeng Yang ET AL: "Isolation and culture of neural crest stem cells from human hair follicles", Journal of visualized experiments : JoVE, 1 January 2013 (2013-01-01), XP055198880, United States DOI: 10.3791/3194 Retrieved from the Internet: URL:http://www.ncbi.nlm.nih.gov/pubmed/236 08752
- LEE GABSANG ET AL: "Derivation of neural crest cells from human pluripotent stem cells", NATURE PROTOCOLS, NATURE PUBLISHING GROUP, GB, vol. 5, no. 4, 1 April 2010 (2010-04-01), pages 688-701, XP009185090, ISSN: 1750-2799, DOI: 10.1038/NPROT.2010.35 [retrieved on 2010-03-18] cited in the application
- COGHLAN M P ET AL: "SELECTIVE SMALL MOLECULE INHIBITORS OF GLYCOGEN SYNTHASE KINASE-3 MODULATE GLYCOGEN METABOLISM AND GENE TRANSCRIPTION", CHEMISTRY AND BIOLOGY, CURRENT BIOLOGY, LONDON, GB, vol. 7, no. 10, 1 January 2000 (2000-01-01), pages 793-803, XP001057051, ISSN: 1074-5521, DOI: 10.1016/S1074-5521(00)00025-9
- Christof Niehrs: "The complex world of WNT receptor signalling", Nat Rev Mol Cell Biol., vol. 13, no. 12, 1 December 2012 (2012-12-01), pages 767-779, XP055397737, ISSN: 1471-0072, DOI: 10.1038/nrm3470
- Anonymous: "KEGG PATHWAY: Wnt signaling pathway - Homo sapiens (human)", , 19 May 2017 (2017-05-19), XP055397739, Retrieved from the Internet: URL:http://www.genome.jp/kegg-bin/show_pat hway?hsa04310 [retrieved on 2017-08-10]
- Michael Kahn: "Can we safely target the WNT pathway?", Nature Reviews. Drug Discovery, vol. 13, no. 7, 1 July 2014 (2014-07-01), pages 513-532, XP055292587, GB ISSN: 1474-1776, DOI: 10.1038/nrd4233

## Description

### [Technical Field]

This invention relates to a method of producing skin-derived precursor cells.

### [Background Art]

Regenerative medicine has become a focus of attention as a medical therapy enabling regeneration of cells, tissues or organs damaged by diseases, accidents or other causes, and restoration of their lost functions. At clinical venues where regenerative medicine is being practiced, various therapies are being tried using artificially cultured cells or tissues for regeneration of cells, tissues and organs lost by surgical treatment, accidents or the like. Moreover, hair follicle regeneration technologies are highly significant in terms of enhancing quality of life (QOL) from the aspect of appearance (the social side), health aspects or the like.

Skin-derived precursor cells (hereinafter also referred to as "SKPs") are known as one source of cells for artificial culture of cells and tissues. SKPs are cells present in dermal papilla that are capable of differentiating into neurons, glial cells (neuroglia cells), smooth muscle cells, adipocytes, osteocytes, dermal fibroblasts, dermal papilla cells or the like. As such, SKPs are cells that fulfill an important function in maintaining a dermal environment, tissue repair, hair follicle formation or the like (see Non-Patent Literatures 1 and 2).

At clinical venues engaged in regenerative medicine, therefore, a need is felt for development of a method of efficiently obtaining SKPs in the large numbers required for regeneration of cells, tissues and organs lost as a result of surgical treatment, accidents or other causes. Further, such a method of efficiently obtaining SKPs in large numbers is desired also for regeneration of the hair follicles that contribute to enhancement of QOL.

Specific examples of methods of obtaining SKPs that have been reported so far include a method of collecting and culturing SKPs as floating human or non-human animal cell aggregates (for example, see Non-Patent Literature 1), and a method of producing SKPs from cells subjected to adhesion culture from human or non-human animal cells (for example, see Non-Patent Literature 3).

### [Citation List]

### [Non Patent Literature]

[NPL 1] Jean G. Toma, et al., Nature Cell Biology, vol. 3, p. 778-784 (2001)
[NPL 2] J. Biemaskie, et al., Cell Stem Cell, vol. 5, p. 610-623 (2009)
[NPL 3] Rebecca P. Hill, et al., PLoS One., vol. 7(11), p. e50742 (2012)

### [Summary of Invention]

The present invention relates to a method of producing SKPs, comprising culturing human-derived pluripotent stem cells in a differentiation-inducing medium containing an agonist of Wnt signaling to differentiate the above-described pluripotent stem cells into SKPs, wherein a basal medium of the differentiation-inducing medium is a D-MEM/Ham's F12 medium, wherein the differentiation-inducing medium further comprises B-27 supplement and at least one nutritional factor selected from the group consisting of epidermal growth factor and basic fibroblast growth factor, and wherein Wnt signaling is a series of actions to enhance nuclear localization of beta-catenin to exhibit a function as a transcription factor.

Moreover, the present invention relates to the use of a differentiation-inducing medium for differentiating human-derived pluripotent stem cells into SKPs, containing an agonist of Wnt signaling as a differentiation-inducing promoter, wherein Wnt signaling is a series of actions to enhance nuclear localization of beta-catenin to exhibit a function as a transcription factor.

Further, the present invention relates to the use of an agonist of Wnt signaling as a differentiation-inducing promoter for differentiating human-derived pluripotent stem cells into SKPs, containing an agonist of Wnt signaling as an active ingredient, wherein Wnt signaling is a series of actions to enhance nuclear localization of beta-catenin to exhibit a function as a transcription factor.

Other and further features and advantages of the invention will appear more fully from the following description, appropriately referring to the accompanying drawings.

### [Brief Description of Drawings]

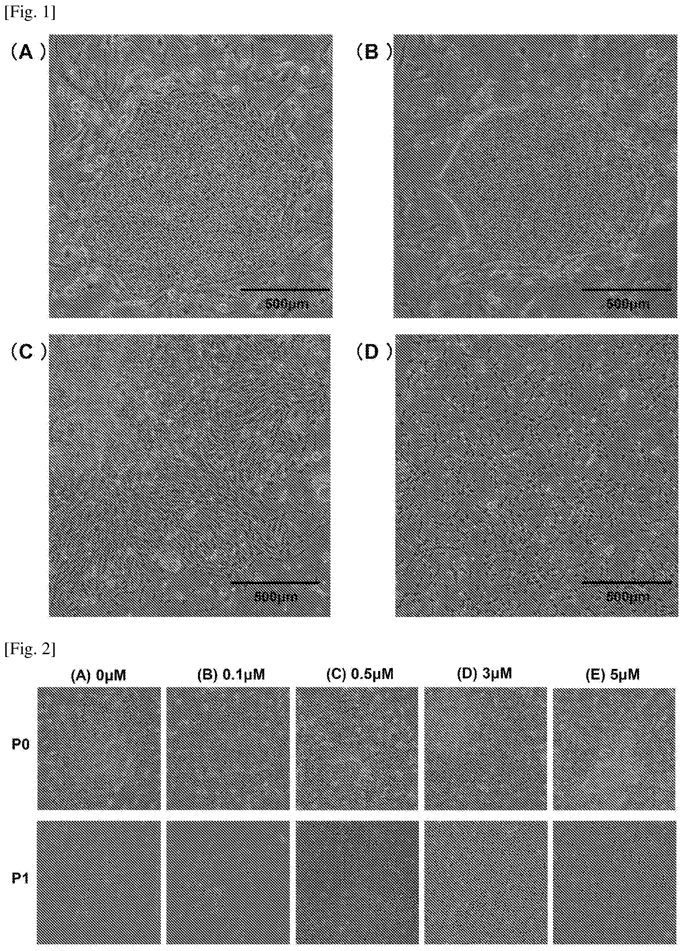

Fig 1(A) shows a microphotograph of human induced pluripotent stem cells (hereinafter also referred to as "iPS cells"), Fig. 1(B) shows a microphotograph of human iPS cell-derived neural crest stem cells, Fig. 1(C) shows a microphotograph of SKPs obtained by culturing human iPS cell-derived neural crest stem cells in a differentiation-inducing medium containing an agonist of Wnt signaling, and Fig. 1(D) shows a microphotograph of cells produced by passage-culturing SKPs obtained by culturing human iPS cell-derived neural crest stem cells in a differentiation-inducing medium containing an agonist of Wnt signaling.
Fig. 2 shows a microphotograph of SKPs differentiated from human iPS cell-derived neural crest stem cells when differentiation was induced by culturing under different concentrations of an agonist of Wnt signaling. Fig. 2(A) shows a microphotograph of cells produced in the case of culturing in a culture medium to which no agonist (0 micromolar) was added, Fig. 2 (B) shows a microphotograph of cells produced in the case of culturing in a culture medium to which the agonist was added at a concentration of 0.1 micromolar, Fig. 2(C) shows a microphotograph of cells produced in the case of culturing in a culture medium to which the agonist was added at a concentration of 0.5 micromolar, Fig. 2(D) shows a microphotograph of cells produced in the case of culturing in a culture medium to which the agonist was added at a concentration of 3 micromolars, and Fig. 2(E) shows a microphotograph of cells produced in the case of culturing in a culture medium to which the agonist was added at a concentration of 5 micromolars. Here, microphotographs of the cells before passage culture are designated "P0", and microphotographs of the cells after passage culture are designated "P 1 ".

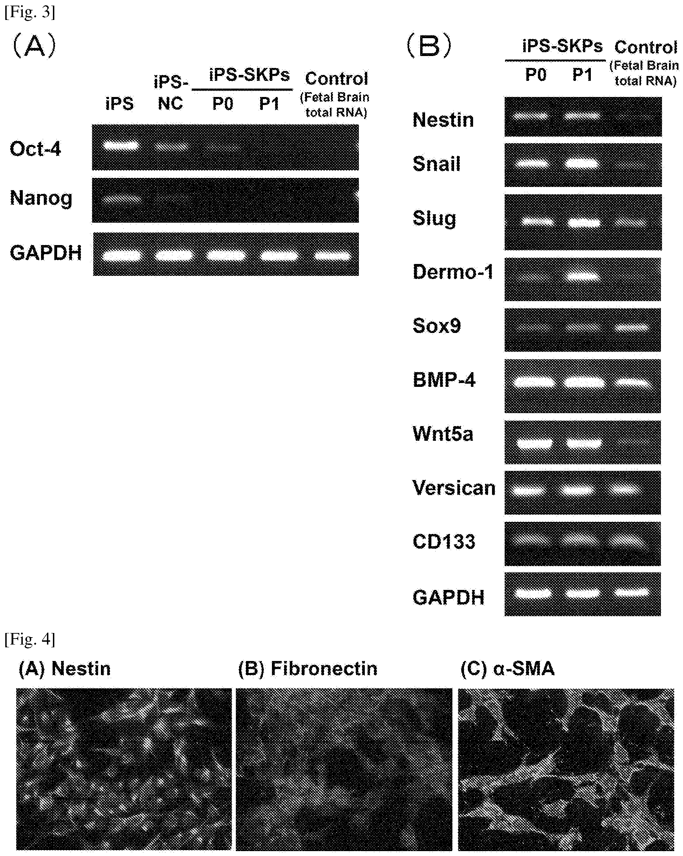

Fig. 3(A) shows electrophoresis photographs in which gene expression of human iPS cells when undifferentiated was compared between before and after differentiation induction into SKPs, and Fig. 3(B) shows electrophoresis photographs comparing expression of genes expressed in SKPs after differentiation induction into SKPs.
Fig. 4(A) is a fluorescence microphotograph showing expression of nestin in human iPS cell-derived SKPs after passage culture, Fig. 4(B) is a fluorescence microphotograph showing expression of fibronectin in human iPS cell-derived SKPs after passage culture, and Fig. 4(C) is a fluorescence microphotograph showing expression of alpha-smooth muscle actin (alpha-SMA) in human iPS cell-derived SKPs after passage culture.

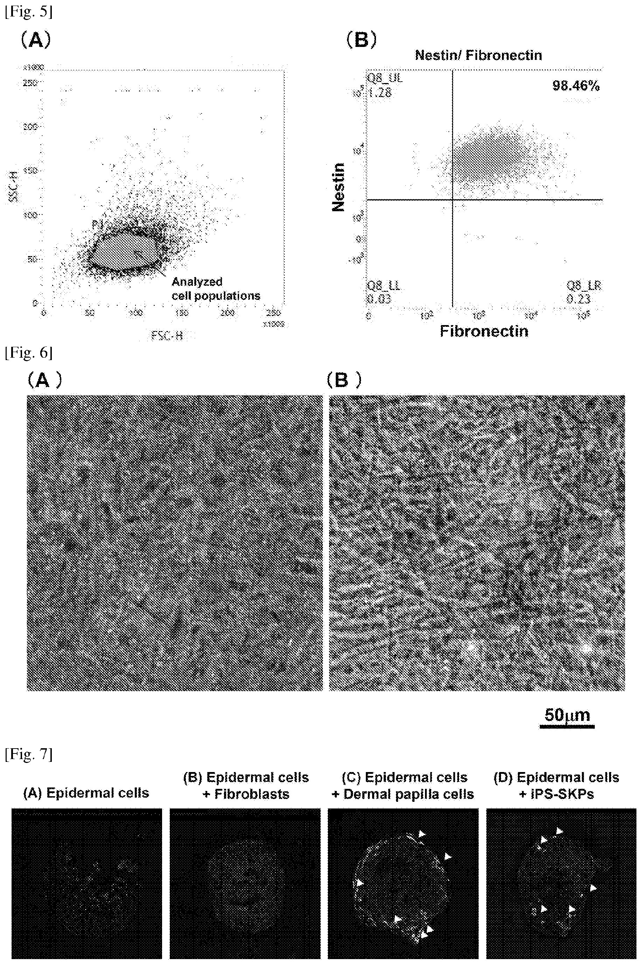

Fig. 5 is a set of diagrams showing the results obtained by performing flow cytometric analysis on SKPs differentiation-induced from iPS cells. Fig. 5(A) shows a diagram obtained by determining cell population of iPS cell-derived SKPs from side scatter (SSC) and forward scatter (FSC) of individual cells, and selecting the cell population (a group of living cells) to be analyzed. Fig. 5(B) is a diagram showing relationship between fluorescence intensity and cell count when the group of cells selected in Fig. 5(A) was stained with an anti-fibronectin antibody and an anti-nestin antibody.
Fig. 6(A) shows a microphotograph of adipocytes obtained by Oil Red O staining of cells produced by additionally subjecting SKPs induced from human iPS cells to 2 weeks of differentiation induction to adipocytes, Fig. 6(B) shows a microphotograph of osteocytes obtained by alkaline phosphatase staining of cells produced by additionally subjecting SKPs induced from human iPS cells to 2 weeks of differentiation induction to osteocytes.
Fig. 7 is a set of microphotographs showing hair follicle induction potency of SKPs produced from human iPS cells. Fig. 7(A) shows a fluorescence microphotograph obtained by immunofluorescence staining, with an anti-trichohyalin antibody, a cell mass obtained by carrying out spheroid culture of only epidermal cells. Fig. 7(B) shows a fluorescence microphotograph obtained by immunofluorescence staining, with an anti-trichohyalin antibody, a cell mass obtained by mixing epidermal cells and fibroblasts, and carrying out spheroid culturing. Fig. 7(C) shows a fluorescence microphotograph obtained by immunofluorescence staining, with an anti-trichohyalin antibody, a cell mass obtained by mixing epidermal cells and dermal papilla cells, and carrying out spheroid culturing. Fig. 7(D) shows a fluorescence microphotograph obtained by immunofluorescence staining, with an anti-trichohyalin antibody, a cell mass obtained by mixing epidermal cells and human iPS cell-derived SKPs, and carrying out spheroid culturing. Arrowheads in the figures indicate expression of trichohyalin.

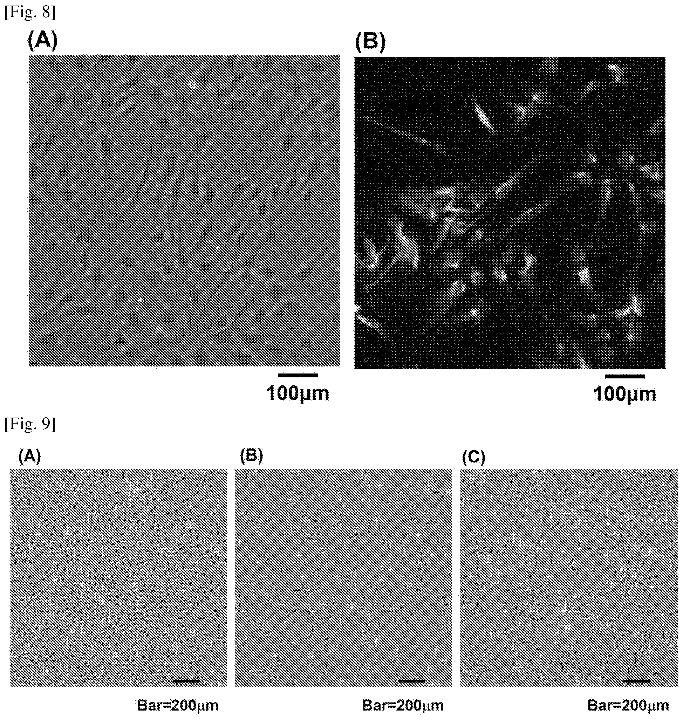

Fig. 8(A) shows a microphotograph of cells produced by additionally subjecting SKPs induced from human iPS cells to 3 weeks of differentiation to Schwann cells. Fig. 8(B) shows a fluorescence microphotograph of cells produced by staining the cells in Fig. 8(A) using an anti-S100-beta antibody.
Fig. 9(A) shows a microphotograph of human iPS cell-derived SKPs before cryopreservation. Fig. 9(B) shows a microphotograph of cells produced by cryopreserving some of the SKPs shown in Fig. 9(A), and then thawing, and culturing the resultant cells for 1 day. Fig. 9(C) shows a microphotograph of cells produced by further carrying out propagation/culturing of the cells shown in Fig. 9(B) for 3 days.

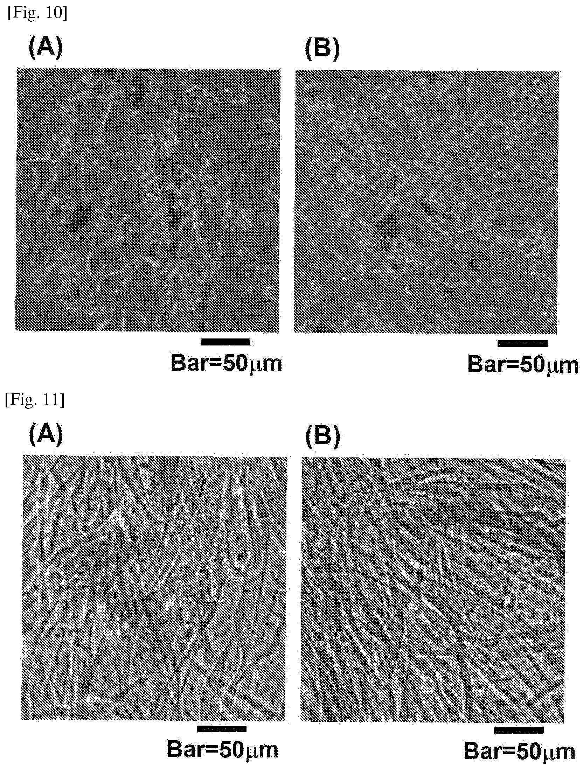

Fig. 10(A) shows a microphotograph of adipocytes stained by Oil Red O staining of cells produced by subjecting human iPS cell-derived SKPs before the cryopreservation to 2 weeks of differentiation induction. Fig. 10(B) shows a microphotograph of adipocytes stained by Oil Red O staining of cells produced by subjecting human iPS cell-derived SKPs after the cyopreservation-thawing to 2 weeks of differentiation induction.
Fig. 11(A) shows a microphotograph of osteocytes stained by alkaline phosphatase staining of cells produced by subjecting human iPS cell-derived SKPs before the cryopreservation to 2 weeks of differentiation induction. Fig. 11(B) shows a microphotograph of osteocytes stained by alkaline phosphatase staining of cells produced by culturing human iPS cell-derived SKPs after the cyopreservation-thawing and then subjecting the resultant SKPs to two weeks of differentiation induction.

### [Description of Embodiments]

As mentioned above, SKPs are cells capable of differentiating into neurons, glial cells, smooth muscle cells, adipocytes, osteocytes, dermal papilla cells or the like. Therefore, SKPs are useful in regenerative medicine and similar fields. As pointed out earlier, the methods described in Non-Patent Literatures 1 and 3 enable production of SKPs that can differentiate into neurons, glial cells, smooth muscle cells, adipocytes, osteocytes, dermal papilla cells or the like. However, the methods described in Non-Patent Literatures 1 and 3 are still far from sufficient in terms of SKPs production efficiency.

Therefore, the present invention is contemplated for providing a method of efficiently producing SKPs capable of differentiating into neurons, glial cells, smooth muscle cells, adipocytes, osteocytes, dermal papilla cells or the like.

Further, the present invention is contemplated for providing a differentiation-inducing medium and a differentiation-inducing promoter that can be preferably used in the above-described method.

In view of the above-described problems, the present inventor continued a diligent study. As a result, the present inventor found that SKPs can be efficiently produced by culturing human-derived pluripotent stem cells in a differentiation-inducing medium containing an agonist of Wnt signaling. The present invention was completed based on this finding.

According to the method of producing SKPs of the present invention, it is possible to efficiently produce SKPs capable of differentiating into neurons, glial cells, smooth muscle cells, adipocytes, osteocytes, dermal papilla cells or the like.

Further, the differentiation-inducing medium and the differentiation-inducing promoter of the present invention can be used in the above-described method.

According to the method of producing SKPs of the present invention, the pluripotent stem cells are cultured using a differentiation-inducing medium containing an agonist of Wnt signaling. Thus, differentiation induction of the pluripotent stem cells to SKPs is performed. Differentiation efficiency of the pluripotent stem cells is improved by culturing pluripotent stem cells in the differentiation-inducing medium containing an agonist of Wnt signaling, and thus SKPs can be efficiently produced.

The present invention is described below in detail by way of a preferred embodiment of the present invention. However, the present invention is not restricted thereto.

"Skin-derived precursor cells (SKPs)" herein means undifferentiated cells having self-renewal potential, and cells having differentiation potential to neurons, glial cells (for example, microglia, astrocytes, oligodendrocytes, ependimocytes, Schwann cells, satellite cells), smooth muscle cells, adipocytes, osteocytes, dermal fibroblasts, dermal papilla cells or the like.

"Pluripotent stem cells" herein means undifferentiated cells having pluripotency allowing differentiation to various tissues that form an adult, and the self-renewal potential. The pluripotent stem cells used in the present invention can be appropriately selected. Specific examples of the pluripotent stem cells include embryonic stem cells (hereinafter, also referred to as "ES cells"), embryonic carcinoma cells (hereinafter, also referred to as "EC cells"), embryonic germ cells (hereinafter, also referred to as "EG cells") and iPS cells. These cells may be produced according to an ordinary method. Alternatively, commercially available cells may be used.

As the human-derived pluripotent stem cells used in the present invention, the ES cells or the iPS cells are preferred, and the iPS cells are further preferred.

Specific examples of the human-derived pluripotent stem cells that can be preferably used in the present invention include human iPS cells established by various methods, such as human iPS cells produced by introducing, into somatic cells such as cutaneous cells, a factor required for maintaining or inducing an undifferentiated state of Oct3/4 genes, Klf4 genes, c-Myc genes and Sox2 genes, and human iPS cells produced by treating somatic cells such as cutaneous cells with a specific compound.

A method of culturing the pluripotent stem cells is described.

In the present invention, the pluripotent stem cells are cultured using a differentiation-inducing medium containing an agonist of Wnt signaling. "Wnt signaling" here is a series of actions to enhance nuclear localization of beta-catenin to exhibit a function as a transcription factor. The Wnt signaling herein includes a series of flows in which a protein called Wnt3A secreted from certain cells, for example, as caused by interaction between the cells, further acts on different cells, and beta-catenin in the cells causes nuclear localization to act as the transcription factor. The series of flows causes a first phenomenon of integrating an organ, taking epithelial-mesenchymal interaction as an example. The Wnt signaling is known to control various kinds of cell functions, such as growth or differentiation of cells, organogenesis, and cytotropism during early development by activating three pathways, a beta-catenin pathway, a PCP pathway and a Ca²⁺ pathway.

In the present invention, a commercially available agonist of Wnt signaling may be used. Alternatively, an agonist of Wnt signaling produced according to an ordinary method may be used. Specific examples of the agonist of Wnt signaling include an aminopyrimidine compound (e.g. CHIR99021 (trade name)), a bis-indolo(indirubin) compound (hereinafter, also referred to as "BIO") (e.g. (2'Z,3'E)-6-bromoindirubin-3'-oxime), an acetoxime compound of BIO (hereinafter, also referred to as "BIO-acetoxime") (e.g. (2'Z,3'E)-6-bromoindirubin-3'-acetoxime), a thiadiazolidine (TDZD) compound (e.g. 4-benzyl-2-methyl-1,2,4-thiadiazolidine-3,5-dione), an oxothiadiazolidine-3-thione compound (e.g. 2,4-dibenzyl-5-oxothiadiazolidine-3-thione), a thienyl alpha-chloromethyl ketone compound (e.g. 2-chloro-1-(4,4-dibromothiophene-2-yl)-ethanone), a phenyl alpha bromomethyl ketone compound (e.g. alpha-4-dibromoacetophenone), a thiazole-containing urea compound (e.g. N-(4-methoxybenzyl)-N'-(5-nitro-1,3-thiazole-2-yl)urea), and a GSK-3 beta peptide inhibitor (e.g. H-KEAPPAPPQSpP-NH₂). The agonist of Wnt signaling used in the present invention includes preferably at least one kind selected from CHIR99021, BIO, NSC693868 (trade name), SB216763 (trade name), SB415286 (trade name) and TWS119 (trade name), further preferably CHIR99021.

A content of the agonist of Wnt signaling contained in the differentiation-inducing medium can be appropriately set up according to culture conditions, kinds of pluripotent stem cells to be used, kinds of agonists of Wnt signalings to be used, or the like, within the range in which the Wnt signaling is activated and cell growth is not broken down. For example, when CHIR99021 is used as the agonist of Wnt signaling, a concentration of the agonist of Wnt signaling in the culture medium is preferably 0.5 micromolar or more, further preferably 2 micromolars or more, and preferably 5 micromolars or less, and further preferably 4 micromolars or less. A concentration range of the agonist of Wnt signaling is preferably 0.5 micromolar to 5 micromolars, and further preferably 2 micromolars to 4 micromolars. Moreover, a concentration of the agonist of Wnt signaling is particularly preferably adjusted to 3 micromolars.

The differentiation-inducing medium used for culture of the pluripotent stem cells can be prepared by adding a predetermined amount of the agonist of Wnt signaling into a culture medium ordinarily used for culturing the stem cells.

A basal medium of the differentiation-inducing medium can be appropriately selected from the culture media ordinarily used for culturing the stem cells. Specific examples include a MEM medium (Minimum Essential Medium), a BME medium (Basal Medium Eagle), an IMDM medium (Iscove's Modified Dulbecco's Medium), a D-MEM medium (Dulbecco's Modified Eagle's Medium), a Ham's medium, a RPMI medium (Roswell Park Memorial Institute medium), a Fischer's medium, and a mixed medium thereof. Among these, a D-MEM/Ham's F12 medium (hereinafter, also referred to simply as "D-MEM/F12") is preferred.

The differentiation-inducing medium used in the present invention may include a serum-containing medium, a serum-free medium or a serum replacement-containing medium. Specific examples of the serum replacement that can be used in the present invention include albumin, transferrin, fatty acid, a collagen precursor, a trace element (e.g. zinc, selenium), a nutritional factor (EGF (epidermal growth factor)), bFGF (basic fibroblast growth factor), B-27 supplement, an N2 supplement, a knockout serum replacement and 2-mercaptoethanol. The differentiation-inducing medium used in the present invention preferably includes a culture medium containing B-27 supplement, and at least one kind of nutritional factor selected from the group consisting of EGF and bFGF, and further preferably a culture medium containing B27 supplement, EGF and bFGF.

Further, if necessary, an ingredient ordinarily used for the culture medium of the stem cells, such as feeder cells, a vitamin, a buffer, inorganic salts, an antibiotic (e.g. penicillin, kanamycin, streptomycin) or the like may be contained in the differentiation-inducing medium.

The differentiation induction from the pluripotent stem cells to SKPs is executed by culturing the cells at a culture temperature suitable for culture of the pluripotent stem cells to be used and for a period sufficient for achieving the differentiation induction to SKPs. For example, when iPS cells are used as the pluripotent stem cells, the cells are preferably cultured for 1 to 20 days.

In the present invention, the differentiation induction may be performed directly from the pluripotent stem cells to SKPs. Alternatively, the pluripotent stem cells may be preliminarily differentiated to neural crest stem cells or mesoderm (preferably neural crest stem cells), and differentiated neural crest stem cells or mesoderm (preferably neural crest stem cells) may be differentiated to SKPs. SKPs can be further efficiently produced by differentiating the pluripotent stem cells to SKPs through the neural crest stem cells or the like. Culture conditions upon differentiating the cells from the neural crest stem cells to SKPs can be appropriately set up. For example, the cells can be efficiently differentiated to SKPs by culturing the neural crest stem cells in the differentiation-inducing medium containing an agonist of Wnt signaling preferably for 3 to 5 days, further preferably 4 days.

Here, "neural crest stem cells" means pluripotent stem cells having self-renewal potential and pluripotency, moving from a dorsal side into the body of neural tube in genesis of a vertebrate to contribute to formation of various tissues. In addition, the differentiation induction from the pluripotent stem cells to the neural crest stem cells and differentiation to the neural crest stem cells can be confirmed according to an ordinary method.

In the present invention, the SKPs differentiated by using the above-described differentiation-inducing medium can be preferably passage-cultured once or twice or more. SKPs can be obtained as a cell population with high purity by carrying out the passage culture.

A method and a frequency of the passage culture of the above-described pluripotent stem cells and SKPs can be appropriately selected from ordinary passage methods according to kinds of cells, a culturing method or the like. For example, with regard to adhesion cultured cells, passage is performed by dilution culture after dissociation of the cells by an enzyme or the like. With regard to suspension cultured cells, the passage is performed by dilution culture.

In the present invention, the passage can be performed by adhesion culture or suspension culture, and the passage is preferably performed under conditions of the adhesion culture.

According to the method of producing SKPs of the present invention, the cells differentiated to SKPs can be produced at a ratio in a level needing neither detachment nor collection. Alternatively, the cells differentiated to SKPs may be detached and collected by an ordinary method. Specific examples of the method of detaching and collecting the cells differentiated to SKPs include a method using a cell sorter and a method using magnetic beads.

The differentiation induction from the pluripotent stem cells or the neural crest stem cells to SKPs can be confirmed by evaluating presence or absence of expression of a protein for developing a function as these cells or genes encoding the protein (hereinafter, also referred to simply as "marker"), or a cell form by observation through a microscope, or the like. For example, the expression of the protein can be confirmed by a method using an antigen-antibody reaction. The expression of the genes can be confirmed by a method using a Northern blot procedure, a reverse transcription-polymeraze chain reaction (RT-PCR), or the like.

When the differentiation induction to SKPs is confirmed by presence or absence of expression of specific genes, as marker genes, Oct-4 genes, Nanog genes, Nestin genes, Snail genes, Slug genes, Dermo-1 genes, Sox9 genes, BMP-4 genes, Wnt-5a genes, Versican genes, CD133 genes or the like can be used.

Among the above-described genes, specific examples of genes that are expressed in iPS cells before differentiation, and the expression decreases by differentiation to other cells include Oct-4 genes and Nanog genes. The differentiation of the iPS cells to SKPs can be confirmed by confirming a decrease in an amount of expression of these genes. Further, specific examples of factors reported to be expressed in SKPs include Nestin genes, Snail genes, Slug genes, Dermo-1 genes, Sox9 genes, BMP-4 genes, Wnt-5a genes and Versican genes. The differentiation of the iPS cells to SKPs can also be confirmed by confirming expression of these genes. Further, specific examples of factors reported to be expressed in both neural crest-derived and mesenchymal system-derived dermal papilla cells include CD133 genes.

When the differentiation induction to SKPs is confirmed by presence or absence of expression of a specific protein, as a marker protein, nestin, alpha-SMA, fibronectin or the like can be used. These are the proteins the expression of which is reported in SKPs, and therefore the differentiation induction to SKPs can be confirmed by performing immunofluorescence staining using an antibody relative to these marker proteins.

As shown in Examples described later, the agonist of Wnt signaling exhibits an action of promoting the differentiation induction of the pluripotent stem cells (preferably the neural crest cells) to SKPs to improve differentiation efficiency of the pluripotent stem cells. Based on this finding, the present invention also provides a differentiation-inducing medium containing the agonist of Wnt signaling as a differentiation-inducing promoter for differentiating the pluripotent stem cells into SKPs. In addition, the present invention also provides a differentiation-inducing promoter for differentiating the pluripotent stem cells into SKPs containing, as an active ingredient, an agonist of Wnt signaling.

Moreover, an agonist of Wnt signaling can be used for a nontherapeutic differentiation induction method for differentiating the pluripotent stem cells to SKPs. "Nontherapeutic" herein means a concept without containing medical practice, namely, without containing procedure practice to a human body by treatment.

A content of the agonist of Wnt signaling in the above-described differentiation-inducing medium and differentiation-inducing promoter can be appropriately set up according to use forms thereof, such as conditions of culture of the pluripotent stem cells.

According to the method of producing SKPs of the present invention, SKPs can be efficiently produced. Then, SKPs obtained by the method of producing SKPs of the present invention are subjected to the differentiation induction to target cells such as neurons, glial cells, smooth muscle cells, adipocytes, osteocytes, dermal fibroblasts or dermal papilla cells, thereby allowing efficient production of these cells.

A culture method, a composition of the culture medium, a differentiation induction method or a passage-culture method upon allowing differentiation induction of SKPs to target cells can be appropriately set up according to an ordinary method.

Moreover, the differentiation to target cells can also be confirmed according to an ordinary method. For example, when SKPs are differentiated to the adipocytes, the differentiation to the adipocytes can be confirmed by staining an intracellular lipid by an Oil Red O staining method, and confirming presence or absence of staining. When SKPs are differentiated to the osteocytes, the differentiation to the osteocytes can be confirmed by staining the cells by an alkaline phosphatase staining method, and confirming presence or absence of staining. When SKPs are differentiated to the dermal papilla cells, potency allowing induction of hair follicle-like keratinization in epithelial cells by an interaction with the epithelial cells, more specifically, a function as the dermal papilla cells can be confirmed by evaluating presence or absence of expression of the marker protein such as trichohyalin by an immunofluorescence staining method. When SKPs are differentiated to the Schwann cells being one kind of glial cells, the differentiation to the Schwann cells can be confirmed by immunofluorescence staining the cells using an anti-S100-beta antibody, and confirming presence or absence of staining.

The cells further differentiated from SKPs can be separated and collected depending on a kind of each cell according to an ordinary method.

The target cells differentiated from SKPs, such as the neurons, the glial cells, the smooth muscle cells, the adipocytes, the osteocytes, the dermal fibroblasts and the dermal papilla cells, as obtained by the present invention, can be preferably used for regeneration of cells, tissues or organs lost by surgical treatment, casualties or the like, regeneration of hair follicles, or the like.

With regard to the embodiments described above, also disclosed by the present invention includes a method of producing cells described below, a differentiation-inducing medium, a differentiation-inducing promoter, use described below, and a method described below.
<1> A method of producing SKPs, comprising culturing human-derived pluripotent stem cells in a differentiation-inducing medium containing an agonist of Wnt signaling to differentiate the pluripotent stem cells into SKPs wherein a basal medium of the differentiation-inducing medium is a D-MEM/Ham's F12 medium,
   wherein the differentiation-inducing medium further comprises B-27 supplement, and at least one nutritional factor selected from the group consisting of epidermal growth factor and basic fibroblast growth factor, and wherein Wnt signaling is a series of actions to enhance nuclear localization of beta-catenin to exhibit a function as a transcription factor.
<2> The producing method described in the above item <1>, wherein the pluripotent stem cells are iPS cells.
<3> The producing method described in the above item <1> or <2>, wherein the pluripotent stem cells are neural crest stem cells derived from pluripotent stem cells.
<4> The producing method described in the above item <3>, wherein the neural crest stem cells derived from the pluripotent stem cells are cultured in the differentiation-inducing medium for 3 to 5 days, preferably for 4 days, to differentiate the cells into SKPs.
<5> The producing method described in any one of the above items <1> to <4>, wherein the agonist of Wnt signaling is at least one selected from the group consisting of CHIR99021, BIO, NSC693868, SB216763, SB415286, and TWS119; preferably CHIR99021.
<6> The producing method described in the above item <5>, wherein the content of the CHIR99021 in the differentiation-inducing medium is preferably 0.5 micromolar or more, and more preferably 2 micromolars or more; preferably 5 micromolars or less, and more preferably 4 micromolars or less; or preferably from 0.5 micromolar to 5 micromolars, more preferably from 2 micromolars to 4 micromolars, and particularly preferably 3 micromolars.
<7> The producing method described in any one of the above items <1> to <6>, wherein differentiation into SKPs is performed under conditions of adhesion culture.
<8> The producing method described in any one of the above items <1> to <7>, wherein SKPs differentiated using the differentiation-inducing medium are passage-cultured one or more times.
<9> The producing method described in any one of the above items <1> to <8>, wherein cells differentiated into SKPs are separated and collected by an ordinary method such as a method using a cell sorter, a method using magnetic beads, or the like.
<10> A method of producing target cells, wherein SKPs produced by the producing method described in any one of the above items <1> to <9> are further differentiated into the target cells.
<11> The method described in the above item <10>, wherein the target cells include any cells selected from the group consisting of neurons, glial cells, smooth muscle cells, adipocytes, osteocytes, dermal fibroblasts and dermal papilla cells, preferably any cells selected from the group consisting of adipocytes, osteocytes, glial cells and dermal papilla cells.
<12> Use of a differentiation-inducing medium for differentiating human-derived pluripotent stem cells into SKPs, containing an agonist of Wnt signaling as a differentiation-inducing promoter, wherein Wnt signaling is a series of actions to enhance nuclear localization of beta-catenin to exhibit a function as a transcription factor.
<13> The use described in the above item <12>, wherein the agonist of Wnt signaling is at least one selected from the group consisting of CHIR99021, BIO, NSC693868, SB216763, SB415286, and TWS119; preferably CHIR99021.
<14> The use described in the above item <12> or <13>, wherein a basal medium of the differentiation-inducing medium is a D-MEM/F12 medium.
<15> The use described in any one of the above items <12> to <14>, wherein the medium further contains B-27 supplement, and at least one nutritional factor selected from the group consisting of EGF and bFGF; preferably B-27 supplement, EGF and bFGF.
<16> The use described in any one of the above items <12> to <15>, wherein the medium further contains an antibiotic; preferably at least one antibiotic selected from the group consisting of penicillin, kanamycin and streptomycin; more preferably penicillin and streptomycin.
<17> Use of an agonist of Wnt signaling as a differentiation-inducing promoter for differentiating human-derived pluripotent stem cells into SKPs, wherein Wnt signaling is a series of actions to enhance nuclear localization of beta-catenin to exhibit a function as a transcription factor.
<18> Use of an agonist of Wnt signaling in the manufacture of a differentiation-inducing promoter for differentiating human-derived pluripotent stem cells into SKPs is also disclosed.
<19> A method of using an agonist of Wnt signaling as a differentiation-inducing promoter for differentiating human-derived pluripotent stem cells into SKPs is also disclosed.
<20> An agonist of Wnt signaling for use in the method of differentiating human-derived pluripotent stem cells into SKPs is also disclosed.
<21> Use of an agonist of Wnt signaling for a nontherapeutic differentiation induction method for differentiating human-derived pluripotent stem cells into SKPs is also disclosed.
<22> A method of performing differentiation induction of human-derived pluripotent stem cells into SKPs, using an agonist of Wnt signaling is also disclosed.
<23> The use or method described in any one of the above items <17> to <22>, wherein the agonist of Wnt signaling is at least one selected from the group consisting of CHIR99021, BIO, NSC693868, SB216763, SB415286, and TWS119; preferably CHIR99021.
<24> The use or method described in any one of the above items <17> to <23>, wherein the pluripotent stem cells are ES cells or iPS cells, preferably iPS cells.

### [Examples]

Hereinafter, the present invention will be described more in detail with reference to Examples, but the present invention is not limited thereto.

### Test Example 1 Passage-culture of iPS cells

### (1) Human iPS cells

As pluripotent stem cells, human-derived iPS cells (trade name: Clone 201B7, passage number: 24, purchased from iPS Academia Japan, Inc.) were used. The above-described iPS cells were obtained by introducing 4 kinds of genes (Oct3/4 genes, Sox2 genes, Klf4 genes, c-Myc genes) into human dermal fibroblasts using a retrovirus vector.

### (2) Preparation of feeder cells

As feeder cells used for culture of the above-described iPS cells, SNL76/7 cells (mouse embryonic fibroblast lines, manufactured by CELL BIOLABS, Inc.) produced by the following method were used.

The SNL76/7 cells were cultured in a D-MEM medium (catalog number: 11965-092, manufactured by Life Technologies Corporation) containing 7% by mass of fetal bovine serum (catalog number: SH30070.03E, manufactured by HyClone Laboratories, Inc.) and penicillin/streptomycin (catalog number: 15140-122, 50U, 50 microgram/mL, manufactured by Life Technologies Corporation). Then, confluent cells were treated with Mitomycin-C (trade name, concentration: 0.012 mg/mL, manufactured by Kyowa Hakko Kirin Co., Ltd.) for 2 hours, and detached by 0.25% trypsin/ethylenediaminetetraacetic acid. In a cell culture dish coated with 0.1% of gelatin (catalog number: G1890, manufactured by Sigma-Aldrich Corporation), detached cells were seeded to be 1 times 10⁶ cells/100 mm dish. After 24 hours, cells adhered on the cell culture dish were used as the feeder cells.

### (3) Culture of human iPS cells

As a culture medium for human iPS cells (hES medium), a D-MEM/F12 medium (D6421, manufactured by Sigma-Aldrich Corporation) containing a serum replacement (catalog number: 10828-028, 20% by mass, manufactured by Life Technologies Corporation), L-glutamine (catalog number: 25030-081, 2 mM, manufactured by Life Technologies Corporation), nonessential amino acid (catalog number: M7145, 0.1 mM, manufactured by Sigma-Aldrich Corporation), 2-mercaptoethanol (catalog number: 21985-023, 0.1 mM, manufactured by Life Technologies Corporation), penicillin/streptomycin (catalog number: 15140-122, 50U, 50 microgram/mL, manufactured by Life Technologies Corporation) and bFGF (catalog number: 064-04541, 4 ng/mL, manufactured by Wako Pure Chemical Industries, Ltd.) was prepared. Human iPS cells were cultured using this culture medium, at 37 degrees in an incubator having 5% CO₂, according to the method described in Cell, 131, pp. 861-872 (2007). Medium replacement was carried out every day.

Then, 80 to 90% confluent iPS cells were treated with a dissociation enzyme (catalog number: RCHETP002, manufactured by ReproCELL Inc.), and iPS cell colonies were detached. The detached colonies were broken in a suitable size by pipetting, and the above-described SNL feeder cells treated with Mitomycin-C were seeded in a preliminary arranged culture, and the iPS cells were cultured at 37 degrees in an incubator having 5% CO₂. Medium replacement was carried out every day.

### Test Example 2 Induction of differentiation from human iPS cell-derived neural crest stem cells to SKPs

### (1) Induction of human iPS cell-derived neural crest stem cells

Based on the method described in Nature protocols, 5, pp. 688-701 (2010) or Cell reports, 3, pp. 1140-1152 (2013), the iPS cells subjected to the passage culture in Test Example 1 were cultured in a culture medium prepared by adding noggin (catalog number: 6057-NG-100/CF, 500 ng/mL, manufactured by R&D Systems, Inc.) and/or SB431542 (catalog number: 1614, 10 micromolars, manufactured by TOCRIS Bioscience) to a hES medium (-) bFGF for 5 days to 2 weeks to induce differentiation from human iPS cells to neural crest stem cells.

### (2) Induction of differentiation from human iPS cell-derived neural crest stem cells to SKPs

The above-described iPS cell-derived neural crest stem cells were cultured in a D-MEM/F12 medium (catalog number: 10565-018, manufactured by Life Technologies Corporation) containing B-27 supplement (catalog number: 17504-044, 2% by mass, manufactured by Life Technologies Corporation), EGF (catalog number: 336-EG-200, 20 ng/mL, manufactured by R&D Systems, Inc.), bFGF (catalog number: 064-04541, 40 ng/mL, manufactured by Wako Pure Chemical Industries, Ltd.), penicillin/streptomycin (catalog number: 15140-122, 50U, 50 microgram/mL, manufactured by Life Technologies Corporation) and 0 micromolar to 5 micromolars of CHIR99021 (catalog number: 13122, manufactured by Cayman Chemical Company). Culture was carried out for 3 to 5 days to induce differentiation from human iPS cell-derived neural crest stem cells to SKPs, and cells differentiated to SKPs were subjected to the passage culture using a culture cell dissociation enzyme (trade name: Accutase, catalog number: 561527, manufactured by BD Biosciences, Inc.), and culture was further carried out in a D-MEM/F12 medium containing B27 supplement (2% by mass), EGF (20 ng/mL), bFGF (40 ng/mL) and penicillin/streptomycin (50U, 50 microgram/mL).

With regard to the thus-obtained SKPs, Fig. 1 shows microphotographs showing an aspect of differentiation to SKPs from human iPS cells before being differentiated to SKPs. In addition, Fig 1(A) shows a microphotograph of human iPS cells, Fig. 1(B) shows a microphotograph of human iPS cell-derived neural crest stem cells, Fig. 1(C) shows a microphotograph of SKPs obtained by culturing human iPS cell-derived neural crest stem cells in a differentiation-inducing medium containing an agonist of Wnt signaling (CHIR99021) in an amount of 3 micromolars, and Fig. 1(D) shows a microphotograph of cells produced by passage-culturing SKPs obtained by culturing human iPS-derived neural crest stem cells in a differentiation-inducing medium containing an agonist of Wnt signaling (CHIR99021) in an amount of 3 micromolars (magnification: 40 times for all).

As shown in Fig. 1(A), the human iPS cells grew in a colony form, abundance of nuclei was high and cytoplasm was small. In contrast, as shown in Fig. 1(D), human iPS cell-derived SKPs after the passage-culturing were not in the colony form, were cultured in a state of individual cells, and existence of clear cytoplasm was recognized. Accordingly, it was confirmed that human iPS cell-derived SKPs were grown by carrying out the passage culture of the cells differentiated to SKPs by the above-described method.

Next, Fig. 2 shows a microphotograph of SKPs differentiated from human iPS cell-derived neural crest stem cells when differentiation was induced by culturing under different concentrations of an agonist of Wnt signaling (CHIR99021). Fig. 2(A) shows a microphotograph of cells produced in the case of culturing in a culture medium to which no CHIR99021 (0 micromolar) was added, Fig. 2(B) shows a microphotograph of cells produced in the case of culturing in a culture medium to which CHIR99021 was added at a concentration of 0.1 micromolar, Fig. 2(C) shows a microphotograph of cells produced in the case of culturing in a culture medium to which CHIR99021 was added at a concentration of 0.5 micromolar, Fig. 2(D) shows a microphotograph of cells produced in the case of culturing in a culture medium to which CHIR99021 was added at a concentration of 3 micromolars, and Fig. 2(E) shows a microphotograph of cells produced in the case of culturing in a culture medium to which CHIR99021 was added at a concentration of 5 micromolars. Here, microphotographs of the cells before passage culture are designated "P0", and microphotographs of the cells after passage culture are designated "P1".

As shown in Fig. 2, an aspect was observed in which a larger amount of the cells differentiated to SKPs was migrated in a peripheral area of the colony when CHIR99021 was added in a concentration of 0.5 micromolar to 5 micromolars even before the passage culture.

In addition, in a stage before the passage culture, cells other than the cells induced to SKPs also were still contained. Consequently, when the passage culture of SKPs was selectively carried out, an aspect was observed in which a larger amount of the cells differentiated to SKPs grew when CHIR99021 was added in a concentration of 0.5 micromolar to 5 micromolars.

Accordingly, it was confirmed that SKPs can be efficiently produced in a large amount by culturing the pluripotent stem cells in the differentiation-inducing medium containing the agonist of Wnt signaling.

### Test Example 3 Identification of human iPS cell-derived SKPs (1)

With regard to the iPS cells before the differentiation induction (hereinafter, also referred to as "iPS"), the iPS cell-derived neural crest stem cells (hereinafter, also referred to as "iPS-NC"), the iPS cell-derived SKPs before passage culture (hereinafter, also referred to as "iPS-SKPs-P0") and the iPS cell-derived SKPs after the passage culture (hereinafter, also referred to as "iPS-SKPs-P1") as obtained in Test Example 2, states of expression of genes shown in Table 1 below were analyzed by the following method according to an RT-PCR method using primers having nucleotide sequences shown in Table 1 below, and the resultant SKPs were identified.

**Table 1**

| Gene | Sense Primer (5'-3') | Anti-Sense Primer (5'-3') |
|---|---|---|
| GAPDH | cggagtcaacggatttggtcg (SEQ ID NO: 1) | agccttctccatggtggtgaa (SEQ ID NO: 2) |
| Oct-4 | cgaaagagaaagcgaaccag (SEQ ID NO: 3) | gtgaagtgagggctcccata (SEQ ID NO: 4) |
| Nanog | cagaaggcctcagcacctac (SEQ ID NO: 5) | gcctccaagtcactggcag (SEQ ID NO: 6) |
| Nestin | cagcgttggaacagaggttg (SEQ ID NO: 7) | gctggcacaggtgtctcaag (SEQ ID NO: 8) |
| Snail | accgcctcgctgccaatgct (SEQ ID NO: 9) | gtgcatcttgagggcaccca (SEQ ID NO: 10) |
| Slug | catctttggggcgagtgagtcc (SEQ ID NO: 11) | cccgtgtgagttctaatgtgtc (SEQ ID NO: 12) |
| Dermo-1 | gcaagaagtcgagcgaagatg (SEQ ID NO: 13) | ggcaatggcagcatcattcag (SEQ ID NO: 14) |
| Sox9 | gtcagccaggtgctcaaagg (SEQ ID NO: 15) | acttgtaatccgggtggtcc (SEQ ID NO: 16) |
| BMP-4 | ttctgcagatgtttgggctgc (SEQ ID NO: 17) | agagccgaagctctgcagag (SEQ ID NO: 18) |
| Wnt-5a | ggatggctggaagtgcaatg (SEQ ID NO: 19) | acacaaactggtccacgatc (SEQ ID NO: 20) |
| Versican | acgatgcctactttgccacc (SEQ ID NO: 21) | tagtgaaacacaaccccatcc (SEQ ID NO: 22) |
| CD133 | atggccctcgtactcggctc (SEQ ID NO: 23) | Cacgcggctgtaccacatag (SEQ ID NO: 24) |

RNA was extracted from each sample using RNeasy Mini kit (catalog number: 74104, manufactured by QIAGEN N.V.). A concentration of each extracted total RNA was measured and a reverse transcription reaction was carried out using a predetermined amount of total RNA and High capacity RNA-to-cDNA Kit (catalog number: 4387406, manufactured by Applied Biosystems, Inc.). Similar operation was performed using human Fetal Brain total RNA (catalog number: 636526, manufactured by Clontech Laboratories, Inc.) as a control.

As a template, 1 microliter of the thus-obtained cDNA sample was used, and PCR was carried out using the above-described primers in 50 microliters of system. An enzyme used here was KOD-Plus-Ver. 2 (catalog number: KOD-211, manufactured by TOYOBO Co., Ltd.), and the PCR was carried out under a reaction protocol of applying 94 degrees, 2 min, and applying 25 to 35 cycles in which (98 degrees, 10 seconds; 63 degrees, 30 seconds; 68 degrees, 30 seconds) was taken as one cycle. Moreover, as a template, human fetal brain-derived RNA (catalog number: 636526, manufactured by Clontech Laboratories, Inc.) was used as a positive control of the PCR, and the PCR was carried out in a similar manner.

Then, electrophoresis was conducted on 5 microliters of the reaction mixture at 100 V by using a 1.5% agarose gel (catalog number: 50071, manufactured by Takara Bio Inc.)/TBE buffer (catalog number: 46510-78, manufactured by Kanto Chemical Co., Inc.). GAPDH was used as a control in experiments as a whole.

Fig. 3(A) and (B) show the results of electrophoresis. Here, Fig. 3(A) shows a gene expression change of a gene important for maintaining an undifferentiated state of human iPS cells, and Fig. 3(B) shows gene expression specific to SKPs.

As shown in Fig. 3(A), in association with the differentiation induction to SKPs, reduction was caused in expression of a marker to be expressed in undifferentiated cells that are not differentiated to SKPs. Further, as shown in Fig. 3(B), expression of genes specific to SKPs was detected by the differentiation induction to SKPs. Accordingly, it was confirmed that the cells subjected to the differentiation induction by the above-described method were SKPs. Further, when expression of the genes specific to SKPs was compared between iPS-SKPs-PO and iPS-SKPs-P1, tendency of higher expression in iPS-SKPs-P1 in comparison with iPS-SKPs-PO was recognized with regard to Snail genes, Slug genes, Dermo-1 genes, Sox9 genes and CD133 genes (see Fig. 3(B)). Here, as shown in Fig. 3(B), expression of GAPDH genes being an internal standard was constant between iPS-SKPs-PO and iPS-SKPs-P1, which shows that a ratio of cells that express the genes specific to SKPs increased by carrying out the passage culture for the cells differentiated to SKPs, and human iPS cell-derived SKPs were obtained as a cell population with higher purity.

### Test Example 4 Identification of human iPS cell-derived SKPs (2)

With regard to human iPS cell-derived SKPs after the passage culture obtained in Test Example 2 described above, immunofluorescence staining was performed by using antibodies shown in Table 2 below, and states of expression of nestin, alpha-SMA and fibronectin were analyzed, and the resultant SKPs were identified.

**Table 2**

| | Target protein | Maker | Detail |
|---|---|---|---|
| Primary antibody | Nestin | Millipore | MAB5326 |
| | α-SMA | Sigma Aldrich | A2547 |
| | fibronectin | Sigma Aldrich | F3648 |
| Secondary antibody | Alexa Fluor 488 goat anti-mouse IgG (H+L) | Life technologies | A11029 |
| | Alexa Fluor 555 donkey anti-rabbit IgG (H+L) | Life technologies | A31572 |

Human iPS cell-derived SKPs after the passage culture obtained in Test Example 2 described above were washed with D-PBS(-), and the resultant SKPs after the passage culture were fixed with 4% paraformaldehyde for 15 minutes. The fixed cells were washed with D-PBS(-), and then treated with a PBS solution of TritonX-100 (concentration: 0.5% by mass) for 5 minutes, the resultant cells were washed again with D-PBS(-), and subjected to blocking using 10% goat serum (catalog number: 426041, manufactured by NICHIREI Corporation) at room temperature for 1 hour. Then, the resultant cells were treated with the primary antibodies (at room temperature, 2 hours) and the secondary antibodies (at room temperature, 1 hour) as shown in Table 2 above, nuclei were stained using 4',6-diamidino-2-phenylindole (DAPI, catalog number: FK045, manufactured by DOJINDO Laboratories), and then embedded. Thus, expression of a marker protein (nestin, fibronectin, alpha-SMA) specific to SKPs was observed under a fluorescence microscope.

Fig. 4 shows the results. Here, Fig. 4(A) is a fluorescence microphotograph showing expression of nestin, Fig. 4(B) is a fluorescence microphotograph showing expression of fibronectin, and Fig. 4(C) is a fluorescence microphotograph showing expression of alpha-SMA (magnification: 400 times).

As shown in Fig. 4, expressions of the marker proteins specific to SKPs were recognized in substantially all cells. From these results, it was confirmed that the cells obtained in Test Example 2 described above were SKPs.

### Test Example 5 Identification of human iPS cell-derived SKPs (3)

With regard to human iPS cell-derived SKPs after the passage culture obtained in Test Example 2 described above, flow cytometric analysis was conducted. Fig. 5 shows the results.

Human iPS cell-derived SKPs after the passage culture obtained in Test Example 2 described above were washed with D-PBS(-), and then the resultant cells were detached using a culture cell dissociation enzyme (trade name: Accutase, catalog number: 561527, manufactured by BD Biosciences, Inc.). The detached cells were fixed at room temperature for 20 minutes using 1 times 10⁶ cells/100 microliters of a sample buffer (trade name: BD Cytofix Buffer, catalog number:554655, manufactured by BD Biosciences Inc.). The fixed cells were washed with a cell permeation washing solution (trade name: BD Phosflow Perm/Wash buffer I, catalog number: 557885, manufactured by BD Biosciences Inc.), and then the resultant cells were treated with the identical buffer at room temperature for 10 minutes. Then, an anti-nestin antibody (catalog number: 561231, manufactured by BD Pharmingen, Inc.), an anti-fibronectin antibody (catalog number: 563100, manufactured by BD Pharmingen, Inc.) and an isotype control (catalog number: 347202, catalog number: 557782, manufactured by BD Biosciences, Inc.) were added at a ratio of 1:20 to allow reaction at room temperature for 30 minutes. The resultant cells were washed with the cell permeation washing solution twice, and dispersed with 500 microliters of PBS, and flow cytometric analysis of the suspension was conducted using BD FACSVerse (manufactured by BD Biosciences, Inc.).

Fig. 5(A) shows a diagram obtained by determining cell population of human iPS cell-derived SKPs from SSC and FSC of individual cells, and selecting the cell population (a group of living cells) to be analyzed. Fig. 5 (B) shows the results of staining the cell population selected in Fig. 5(A) using an anti-fibronectin antibody and an anti-nestin antibody. A vertical axis shows expression intensity (fluorescence intensity obtained by fluorescence staining) of nestin, and a horizontal axis shows expression intensity (fluorescence intensity obtained by fluorescence staining) of fibronectin.

From the results shown in Fig. 5(B), a ratio of the cell count expressing both nestin and fibronectin to the total cell count (cell count in the selected region in Fig. 5(A)) used for the flow cytometric analysis in Fig. 5(B) was calculated. As a result, it was confirmed that 98.46% of cells express nestin and fibronectin (positive for both nestin and fibronectin).

Accordingly, it was confirmed that substantially all cells of the cells subjected to the differentiation induction by the above-described method were SKPs expressing nestin and fibronectin. Moreover, it was confirmed that SKPs were obtained as a cell population with high purity by carrying out passage culture of the cells produced by the above-described method.

### Test Example 6 Induction to adipocytes from human iPS cell-derived SKPs

Human iPS cell-derived SKPs after the passage culture obtained in Test Example 2 described above were seeded at 3 times 10⁵ cells/35 mm dish. After culture for 24 hours, the resultant cells were cultured for 2 weeks in an MEM medium (catalog number: 42360-032, manufactured by Life Technologies Corporation) containing 3-isobutyl-1-methylxanthine (catalog number: 17018, 0.45 nM, manufactured by Sigma-Aldrich Corporation), insulin (catalog number: 13536, 2.07 micromolars, manufactured by Sigma-Aldrich Corporation), dexamethasone (catalog number: D4902, 100 nM, manufactured by Sigma-Aldrich Corporation), rabbit serum (catalog number: R4505, 15%, manufactured by Sigma-Aldrich Corporation) and penicillin/streptomycin (catalog number: 15140-122, 100U, 100 microgram/mL, manufactured by Life Technologies Corporation).

Then, Oil Red O staining of the resultant cells was performed using Oil Red O staining Kit (catalog number: 0843, manufactured by ScienCell Research Laboratories) according to an attached protocol. Fig. 6(A) shows the results (magnification: 200 times).

As shown in Fig. 6(A), a lipid was stained red to allow confirmation of achievement of differentiation of human iPS cell-derived SKPs to adipocytes. Accordingly, it was confirmed that the cells obtained in Test Example 2 described above were SKPs that can be differentiated to the adipocytes.

### Test Example 7 Induction to osteocytes from human iPS cell-derived SKPs

Human iPS cell-derived SKPs after the passage culture obtained in Test Example 2 described above were seeded at 3 times 10 cells/35 mm dish. After culture for 24 hours, the resultant cells were cultured for 2 weeks in an MEM medium (catalog number: 42360-032, manufactured by Life Technologies Corporation) containing dexamethasone (catalog number: D4902, 100 nM, manufactured by Sigma-Aldrich Corporation), beta-glycerophosphate (catalog number: G9422, 10 mM, manufactured by Sigma-Aldrich Corporation), L-Ascorbic acid-2-phosphate (catalog number: A8960, 50 micromolars, manufactured by Sigma-Aldrich Corporation), fetal bovine serum (catalog number: SH30070.03, 10 mass%, manufactured by Hyclone) and penicillin/streptomycin (catalog number: 15140-122, 100 U, 100 microgram/mL, manufactured by Life Technologies Corporation).

Then, alkaline phosphatase staining of the resultant cells was performed using Blue Alkaline Phosphatase substrate Kit (catalog number: SK5300, manufactured by Vector laboratories) according to an attached protocol. Fig. 6(B) shows the results (magnification: 200 times).

As shown in Fig. 6(B), alkaline phosphatase-positive cells were recognized to allow confirmation of achievement of differentiation of human iPS cell-derived SKPs to osteocytes. Accordingly, it was confirmed that the cells obtained in Test Example 2 described above were SKPs that can be differentiated to the osteocytes.

### Test Example 8 Study of hair follicle induction potency of human iPS cell-derived SKPs by spheroid culture

Commercially available normal human epidermal cells (NHEK) (manufactured by Life Technologies Corporation) were subjected passage culture in EpiLife (trade name, manufactured by Life Technologies Corporation), and commercially available normal human dermal papilla cells (manufactured by Cell Applications, Inc.) were subjected to the passage culture in a dermal papilla growth medium (manufactured by TOYOBO Co., Ltd.), at 37degrees under 5% CO₂. Moreover, commercially available normal human adult dermal fibroblasts (manufactured by Kurabo Industries Ltd.) were subjected to the passage culture in a D-MEM medium (catalog number: 11965-092, manufactured by Life Technologies Corporation) containing 5% by mass fetal bovine serum (catalog number: SH30070.03E, manufactured by HyClone Laboratories, Inc.) and penicillin/streptomycin (catalog number: 15140-122, 50U, 50 microgram/mL, manufactured by Life Technologies Corporation). Moreover, as SKPs, human iPS cell-derived SKPs (iPS-SKPs-P1) after the passage culture obtained in Test Example 2 described above were used.

After various kinds of cells were subjected to the passage culture, epidermal cells, fibroblasts, dermal papilla cells and human iPS cell-derived SKPs were mixed by 4 times 10⁴ cells for each, and the resultant mixture was cultured using an AmnioMAX C-100 medium (trade name, manufactured by Life Technologies Corporation) by means of a 96-well nonadhesion round bottom plate (manufactured by CellSeed Inc.). After culture for 7 days, the resultant spheroid was embedded with a frozen tissue embedding agent (trade name: OCT Compound, manufactured by Sakura Finetek Co., Ltd.), and a frozen section having a thickness of 6 micrometers was produced.

With regard to the thus produced frozen section, the sections were fixed with 4% paraformaldehyde for 15 minutes, the fixed sections were washed with D-PBS(-), and subjected to blocking using 10% goat serum (catalog number: 426041, manufactured by NICHIREI Corporation) at room temperature for 1 hour. Then, the resultant sections were treated with an anti-trichohyalin antibody (catalog number: sc-80607, manufactured by Santa Cruz Biotechnology, Inc.) at room temperature for 2 hours, and the resultant sections were washed with D-PBS(-), and then the resultant sections were treated with a secondary antibody (Alexa Fluor 488 goat anti-mouse IgG (H+L), catalog number: A 11029, manufactured by Life Technologies Corporation) at room temperature for 1 hour. The resultant sections were washed with D-PBS(-), and then nuclei were stained using 4',6-diamidino-2-phenylindole (DAPI, catalog number: FK045, manufactured by DOJINDO Laboratories), and then embedded, and thus stainability of trichohyalin was observed under a fluorescence microscope.

Fig. 7 shows the results. Here, Fig. 7(A) shows a fluorescence microphotograph obtained by immunofluorescence staining, with an anti-trichohyalin antibody, a cell mass obtained by carrying out spheroid culture of only epidermal cells. Fig. 7(B) shows a fluorescence microphotograph obtained by immunofluorescence staining, with an anti-trichohyalin antibody, a cell mass obtained by mixing epidermal cells and fibroblasts, and carrying out spheroid culturing. Fig. 7(C) shows a fluorescence microphotograph obtained by immunofluorescence staining, with an anti-trichohyalin antibody, a cell mass obtained by mixing epidermal cells and dermal papilla cells, and carrying out spheroid culturing. Fig. 7(D) shows a fluorescence microphotograph obtained by immunofluorescence staining, with an anti-trichohyalin antibody, a cell mass obtained by mixing epidermal cells and human iPS cell-derived SKPs, and carrying out spheroid culturing. Arrowheads in the figures indicate expression of trichohyalin.

As shown in Fig. 7(A) and (B), no expression of trichohyalin was induced in culture of only the epidermal cells or mixed culture of the epidermal cells and the fibroblasts. In contrast, as shown in Figs. 7(C) and (D), it was confirmed that expression of trichohyalin was induced in mixed culture of the epidermal cells with the dermal papilla cells or the human iPS cell-derived SKPs.

Accordingly, it was confirmed that the cells obtained in Test Example 2 described above had hair follicle induction potency in a manner similar to the dermal papilla cells.

### Test Example 9 Induction to glial cells from human iPS cell-derived SKPs

SKPs after the passage culture obtained in Test Example 2 described above were seeded, using a culture medium for SKPs culture (DMEM/F12 medium (catalog number: 10565-018, manufactured by Life Technologies Corporation) containing 2% B-27 supplement (catalog number: 17504-044, manufactured by Life Technologies Corporation), 20 ng/mL EGF (catalog number: 336-EG-200, manufactured R&D Systems, Inc.), 40 ng/mL bFGF (catalog number: 064-04541, manufactured by Wako Pure Chemical Industries, Ltd.), 50U penicillin and 50 microgram/mL streptomycin (catalog number: 15140-122, manufactured by Life Technologies Corporation), in a culture dish pre-coated with Laminin diluted by 25 times (catalog number: P4707, manufactured by Sigma-Aldrich Corporation) and 0.1 mg/mL Poly-L-lysine (catalog number: L4544, manufactured by Sigma-Aldrich Corporation), at 4.8 times 10⁴ cells/35 mm dish. After culture for 24 hour, the cells were cultured for 2 to 3 weeks in a DMEM:F12 medium (catalog number: 10565-018, manufactured by Life Technologies Corporation) containing 5 micromolars Forskoline (catalog number: F3917, manufactured by Sigma-Aldrich Corporation), 50 ng/mL Heregulin-1-beta (catalog number: 100-03, manufactured by PeproTech, Inc.), 2% N2 supplement (catalog number: 17502-048, manufactured by Life Technologies Corporation) and 1% bovine serum (catalog number: SH30070.03E , manufactured by HyClone Laboratories, Inc.). Medium replacement was carried out once every two to three days.

The resultant cells were washed with D-PBS(-), and fixed with 4% paraformaldehyde for 15 minutes. The fixed cells were washed with D-PBS(-), and then treated with a PBS solution of 0.5% TritonX-100 for 5 minutes, the resultant cells were washed again with D-PBS(-), and subjected to blocking using 10% goat serum (catalog number: 426041, manufactured by NICHIREI Corporation) at room temperature for 1 hour. Then, the resultant cells were treated with a primary antibody (anti-S100-beta antibody, catalog number: S2532, manufactured by Sigma-Aldrich Corporation, at room temperature, 2 hours) and a secondary antibody (Alexa Fluor 488 goat anti-mouse IgG(H+L), catalog number: A11029, manufactured by Life Technologies Corporation, at room temperature, 1 hour). Then, nuclei were stained using DAPI (catalog number: FK045, manufactured by DOJINDO Laboratories), and then embedded, and expression of a marker protein (S100-beta) specific to Schwann cells was observed under a fluorescence microscope.

Fig. 8 shows the results. Here, Fig. 8(A) shows a microphotograph of cells produced by additionally subjecting SKPs induced from human iPS cells to 3 weeks of differentiation to Schwann cells, and Fig. 8(B) shows a fluorescence microphotograph of cells produced by staining the cells in Fig. 8(A) using an anti-S100-beta antibody.

As shown in Fig. 8, S100-beta-positive cells were recognized to allow confirmation of achievement of differentiation of the human iPS cell-derived SKPs to the Schwann cells. Accordingly, it was confirmed that the cells obtained in Test Example 2 described above were SKPs that can be differentiated to the glial cells, such as Schwann cells.

As shown in Test Examples 3 to 9 described above, it was confirmed that the cells obtained in Test Example 2 described above were SKPs. Thus, according to the present invention, SKPs that can be differentiated to the neurons, the glial cells, the smooth muscle cells, the adipocytes, the osteocytes, the dermal papilla cells or the like can be efficiently produced in a large amount.

### Test Example 10 Cryopreservation of human iPS cell-derived SKPs obtained according to the present invention

Then, 1 times 10⁶ cells of SKPs after the passage culture obtained in Test Example 2 described above were suspended into 1 mL of Cell Banker I (catalog number: 248085, manufactured by LSI Medience Corporation), and frozen at -80 degrees. After elapse of 2 to 3 days, frozen cells were preserved in liquid nitrogen.

Preserved cells were thawed, and the thawed cells were cultured in a culture medium for human iPS cell-derived SKPs (DMEM/F12 medium (catalog number: 10565-018, manufactured by Life Technologies Corporation) containing 2% B27 supplement (catalog number: 17504-044, manufactured by Life Technologies Corporation), 20 ng/mL EGF (catalog number: 336-EG-200, manufactured R&D Systems, Inc.), 40 ng/mL bFGF (catalog number: 064-04541, manufactured by Wako Pure Chemical Industries, Ltd.), 50U penicillin and 50 microgram/mL streptomycin (catalog number: 15140-122, manufactured by Life Technologies Corporation).

Fig. 9 shows microphotographs of human iPS cell-derived SKPs before cryopreservation and after thawing of the SKPs. Fig. 9(A) shows a microphotograph of SKPs before cryopreservation; Fig. 9(B) shows a microphotograph of cells produced by cryopreserving some of the SKPs shown in Fig. 9(A), and then thawing, and culturing the resultant cells for 1 day; and Fig. 9(C) shows a microphotograph of cells produced by further carrying out propagation/culturing of the cells shown in Fig. 9(B) for 3 days.

As shown in Fig. 9, it was confirmed that the human iPS cell-derived SKPs grew even if the cells were subjected to freezing and thawing, and culture can be continued. Accordingly, it was shown that the human iPS cell-derived SKPs obtained in the present invention allow the cryopreservation and the passage culture.

### Test Example 11 Induction to adipocytes from post-freeze-thaw iPS cell-derived SKPs

To each of human iPS cell-derived SKPs before the cryopreservation shown in Fig. 9(A) and iPS cell-derived SKPs after being frozen and thawed as shown in Fig. 9(C), as obtained in Test Example 10, induction of differentiation of the cells to adipocytes was performed in a manner similar to the method in Test Example 6. Fig. 10 shows the results. Here, Fig. 10(A) shows a microphotograph of adipocytes stained by Oil Red O staining of cells produced by subjecting human iPS cell-derived SKPs before the cryopreservation to 2 weeks of differentiation induction. Fig. 10(B) shows a microphotograph of adipocytes stained by Oil Red O staining of cells produced by subjecting human iPS cell-derived SKPs after the cyopreservation-thawing to 2 weeks of differentiation induction.

As shown in Fig. 10, in a manner similar to the human iPS cell-derived SKPs before the cryopreservation, a lipid was stained red also in the human iPS cell-derived SKPs after being frozen, thawed, grown and cultured to allow confirmation of achievement of differentiation of the human iPS cell-derived SKPs to the adipocytes. Accordingly, it was shown that the cells obtained in Test Example 2 described above kept differentiation induction potency to the adipocytes even after the cells were subjected to passage and the cryopreservation.

### Test Example 12 Induction to osteocytes from post-freeze-thaw iPS cell-derived SKPs

To each of human iPS-derived SKPs before the cryopreservation shown in Fig. 9(A) and iPS cell-derived SKPs after being frozen and thawed as shown in Fig. 9(C), as obtained in Test Example 10, induction of differentiation of the cells to osteocytes was performed in a manner similar to the method in Test Example 7. Fig. 11 shows the results. Here, Fig. 11(A) shows a microphotograph of osteocytes stained by alkaline phosphatase staining of cells produced by subjecting human iPS cell-derived SKPs before the cryopreservation to 2 weeks of differentiation induction. Fig. 11(B) shows a microphotograph of osteocytes stained by alkaline phosphatase staining of cells produced by culturing human iPS cell-derived SKPs after the cyopreservation-thawing and then subjecting the resultant SKPs to two weeks of differentiation induction.

As shown in Fig. 11, in a manner similar to the human iPS cell-derived SKPs before the cryopreservation, alkaline phosphatase-positive cells were recognized also in the human iPS cell-derived SKPs after being frozen, thawed, grown and cultured to allow confirmation of achievement of differentiation of the human iPS cell-derived SKPs to the osteocytes. Accordingly, it was shown that the cells obtained in Test Example 2 described above kept differentiation induction potency to the osteocytes even after the cells were subjected to passage and the cryopreservation.

## Claims

1. A method of producing skin-derived precursor cells, comprising culturing human-derived pluripotent stem cells in a differentiation-inducing medium containing an agonist of Wnt signaling to differentiate the pluripotent stem cells into skin-derived precursor cells,
wherein a basal medium of the differentiation-inducing medium is a D-MEM/Ham's F12 medium,
wherein the differentiation-inducing medium further comprises B-27 supplement, and at least one nutritional factor selected from the group consisting of epidermal growth factor and basic fibroblast growth factor, and
wherein Wnt signaling is a series of actions to enhance nuclear localization of beta-catenin to exhibit a function as a transcription factor.

2. The method according to Claim 1, wherein the pluripotent stem cells are induced pluripotent stem cells.

3. The method according to Claim 1, wherein the pluripotent stem cells are neural crest stem cells derived from pluripotent stem cells.

4. A method of producing skin-derived precursor cells, comprising culturing human-derived neural crest stem cells in a differentiation-inducing medium containing an agonist of Wnt signaling to differentiate the neural crest stem cells into skin-derived precursor cells, wherein Wnt signaling is a series of actions to enhance nuclear localization of beta-catenin to exhibit a function as a transcription factor.

5. The method according to any one of Claims 1 to 4, wherein skin-derived precursor cells differentiated using the differentiation-inducing medium are passage-cultured one or more times.

6. Use of a medium comprising an agonist of Wnt signaling as a differentiation-inducing promoter for differentiating human-derived pluripotent stem cells into skin-derived precursor cells, wherein Wnt signaling is a series of actions to enhance nuclear localization of beta-catenin to exhibit a function as a transcription factor.

7. The use according to Claim 6, wherein a basal medium of the medium is a D-MEM/Ham's F12 medium.

8. The use according to Claims 6 or 7, wherein the medium further comprises B-27 supplement, and at least one nutritional factor selected from the group consisting of epidermal growth factor and basic fibroblast growth factor.

9. Use of an agonist of Wnt signaling as a differentiation-inducing promoter for differentiating human-derived pluripotent stem cells into skin-derived precursor cells, wherein Wnt signaling is a series of actions to enhance nuclear localization of beta-catenin to exhibit a function as a transcription factor.

## Patentansprüche

1. Verfahren zur Herstellung hautabgeleiteter Vorläuferzellen, umfassend das Züchten von vom Menschen stammender pluripotenter Stammzellen in einem Medium, das Differenzierung induziert, das einen Agonisten der Wnt-Signalgebung enthält, um die pluripotenten Stammzellen in hautabgeleitete Vorläuferzellen zu differenzieren,
wobei ein Basalmedium des Mediums, das Differenzierung induziert, ein D-MEM/Ham's F12-Medium ist,
wobei das Medium, das Differenzierung induziert, des Weiteren B-27-Supplement und mindestens einen Nährfaktor ausgewählt aus der Gruppe bestehend aus epidermalem Wachstumsfaktor und basischem Fibroblasten-Wachstumsfaktor umfasst, und
wobei die Wnt-Signalgebung eine Abfolge von Ereignissen zur Verstärkung der nukleären Lokalisierung von beta-Catenin ist, damit es eine Funktion als Transkriptionsfaktor aufweist.

2. Verfahren nach Anspruch 1, wobei die pluripotenten Stammzellen induzierte pluripotente Stammzellen sind.

3. Verfahren nach Anspruch 1, wobei die pluripotenten Stammzellen Stammzellen der Neuralleiste sind, die von pluripotenten Stammzellen stammen.

4. Verfahren zur Herstellung hautabgeleiteter Vorläuferzellen, umfassend das Züchten von vom Menschen stammenden Stammzellen der Neuralleiste in einem Medium, das Differenzierung induziert, das einen Agonisten der Wnt-Signalgebung enthält, um die Stammzellen der Neuralleiste in hautabgeleitete Vorläuferzellen zu differenzieren, wobei die Wnt-Signalgebung eine Abfolge von Ereignissen zur Verstärkung der nukleären Lokalisierung von beta-Catenin ist, damit es eine Funktion als Transkriptionsfaktor aufweist.

5. Verfahren nach einem der Ansprüche 1 bis 4, wobei unter Verwendung des Mediums, das Differenzierung induziert, differenzierte hautabgeleitete Vorläuferzellen über ein oder mehrere Passagen kultiviert wurden.

6. Verwendung eines Mediums, das einen Agonisten der Wnt-Signalgebung als Promotor, der Differenzierung induziert, enthält, zur Differenzierung von vom Menschen stammenden pluripotenten Stammzellen in hautabgeleitete Vorläuferzellen, wobei die Wnt-Signalgebung eine Abfolge von Ereignissen zur Verstärkung der nukleären Lokalisierung von beta-Catenin ist, damit es eine Funktion als Transkriptionsfaktor aufweist.

7. Verwendung nach Anspruch 6, wobei ein Basalmedium des Mediums ein D-MEM/Ham's F12-Medium ist.

8. Verwendung nach Anspruch 6 oder 7, wobei das Medium des Weiteren B-27-Supplement und mindestens einen Nährfaktor ausgewählt aus der Gruppe bestehend aus epidermalem Wachstumsfaktor und basischem Fibroblasten-Wachstumsfaktor umfasst.

9. Verwendung eines Agonisten der Wnt-Signalgebung als Promotor, der Differenzierung induziert, zur Differenzierung von vom Menschen stammenden pluripotenten Stammzellen in hautabgeleitete Vorläuferzellen, wobei die Wnt-Signalgebung eine Abfolge von Ereignissen zur Verstärkung der nukleären Lokalisierung von beta-Catenin ist, damit es eine Funktion als Transkriptionsfaktor aufweist.

## Revendications

1. Méthode de production de cellules précurseurs dérivées de la peau, comprenant la culture de cellules souches pluripotentes d'origine humaine dans un milieu inducteur de différenciation contenant un agoniste de signalisation Wnt pour différencier les cellules souches pluripotentes en cellules précurseurs dérivées de la peau,
dans laquelle un milieu basal du milieu inducteur de différenciation est un milieu D-MEM/Ham's F12,
dans laquelle le milieu inducteur de différenciation comprend en outre un supplément B-27, et au moins un facteur nutritionnel choisi dans le groupe constitué du facteur de croissance de l'épiderme et du facteur de croissance basique des fibroblastes, et
dans laquelle la signalisation Wnt est une série d'actions permettant d'améliorer la localisation nucléaire de la bêta-caténine afin qu'elle présente une fonction en tant que facteur de transcription.

2. Méthode selon la revendication 1, dans laquelle les cellules souches pluripotentes sont des cellules souches pluripotentes induites.

3. Méthode selon la revendication 1, dans laquelle les cellules souches pluripotentes sont des cellules souches de crête neurale dérivées de cellules souches pluripotentes.

4. Méthode de production de cellules précurseurs dérivées de la peau, comprenant la culture de cellules souches de crête neurale d'origine humaine dans un milieu inducteur de différenciation contenant un agoniste de signalisation Wnt pour différencier les cellules souches de crête neurale en cellules précurseurs dérivées de la peau, dans laquelle la signalisation Wnt est une série d'actions permettant d'améliorer la localisation nucléaire de la bêta-caténine afin qu'elle présente une fonction en tant que facteur de transcription.

5. Méthode selon l'une quelconque des revendications 1 à 4, dans laquelle les cellules précurseurs dérivées de la peau différenciées en utilisant le milieu inducteur de différenciation sont cultivées en passage d'une ou plusieurs fois.

6. Utilisation d'un milieu comprenant un agoniste de signalisation Wnt en tant que promoteur inducteur de différenciation pour différencier les cellules souches pluripotentes d'origine humaine en cellules précurseurs dérivées de la peau, dans laquelle la signalisation Wnt est une série d'actions permettant d'améliorer la localisation nucléaire de la bêta-caténine afin qu'elle présente une fonction en tant que facteur de transcription.

7. Utilisation selon la revendication 6, dans laquelle un milieu basal du milieu est un milieu D-MEM/Ham's F12.

8. Utilisation selon les revendications 6 ou 7, dans laquelle le milieu comprend en outre un supplément B-27 et au moins un facteur nutritionnel choisi dans le groupe constitué du facteur de croissance de l'épiderme et du facteur de croissance basique des fibroblastes.

9. Utilisation d'un agoniste de signalisation de Wnt en tant que promoteur inducteur de différenciation pour différencier les cellules souches pluripotentes d'origine humaine en cellules précurseurs dérivées de la peau, dans laquelle la signalisation Wnt est une série d'actions permettant d'améliorer la localisation nucléaire de la bêta-caténine afin qu'elle présente une fonction en tant que facteur de transcription.


### SEQUENCE LISTING

<110> KAO CORPORATION
<120> Method of producing skin-derived precursor cells
<130> P14-0862WO00
<150> JP 2014-087247
   <151> 2014-04-21
<150> JP 2014-260434
   <151> 2014-12-24
<160> 24
<170> PatentIn version 3.5
<210> 1
   <211> 21
   <212> DNA
   <213> Artificial Sequence
<220>
   <223> Oligonucleotide for amplifying the nucleotide sequence of GAPDH gene
<400> 1
   cggagtcaac ggatttggtc g 21
<210> 2
   <211> 21
   <212> DNA
   <213> Artificial Sequence
<220>
   <223> Oligonucleotide for amplifying the nucleotide sequence of GAPDH gene
<400> 2
   agccttctcc atggtggtga a 21
<210> 3
   <211> 20
   <212> DNA
   <213> Artificial Sequence
<220>
   <223> Oligonucleotide for amplifying the nucleotide sequence of Oct-4 gene
<400> 3
   cgaaagagaa agcgaaccag 20
<210> 4
   <211> 20
   <212> DNA
   <213> Artificial Sequence
<220>
   <223> Oligonucleotide for amplifying the nucleotide sequence of Oct-4 gene
<400> 4
   gtgaagtgag ggctcccata 20
<210> 5
   <211> 20
   <212> DNA
   <213> Artificial Sequence
<220>
   <223> Oligonucleotide for amplifying the nucleotide sequence of Nanog gene
<400> 5
   cagaaggcct cagcacctac 20
<210> 6
   <211> 19
   <212> DNA
   <213> Artificial Sequence
<220>
   <223> Oligonucleotide for amplifying the nucleotide sequence of Nanog gene
<400> 6
   gcctccaagt cactggcag 19
<210> 7
   <211> 20
   <212> DNA
   <213> Artificial Sequence
<220>
   <223> Oligonucleotide for amplifying the nucleotide sequence of Nestin gene
<400> 7
   cagcgttgga acagaggttg 20
<210> 8
   <211> 20
   <212> DNA
   <213> Artificial Sequence
<220>
   <223> Oligonucleotide for amplifying the nucleotide sequence of Nestin gene
<400> 8
   gctggcacag gtgtctcaag 20
<210> 9
   <211> 20
   <212> DNA
   <213> Artificial Sequence
<220>
   <223> Oligonucleotide for amplifying the nucleotide sequence of Snail gene
<400> 9
   accgcctcgc tgccaatgct 20
<210> 10
   <211> 20
   <212> DNA
   <213> Artificial Sequence
<220>
   <223> Oligonucleotide for amplifying the nucleotide sequence of Snail gene
<400> 10
   gtgcatcttg agggcaccca 20
<210> 11
   <211> 22
   <212> DNA
   <213> Artificial Sequence
<220>
   <223> Oligonucleotide for amplifying the nucleotide sequence of Slug gene
<400> 11
   catctttggg gcgagtgagt cc 22
<210> 12
   <211> 22
   <212> DNA
   <213> Artificial Sequence
<220>
   <223> Oligonucleotide for amplifying the nucleotide sequence of Slug gene
<400> 12
   cccgtgtgag ttctaatgtg tc 22
<210> 13
   <211> 21
   <212> DNA
   <213> Artificial Sequence
<220>
   <223> Oligonucleotide for amplifying the nucleotide sequence of Dermo-1 gene
<400> 13
   gcaagaagtc gagcgaagat g 21
<210> 14
   <211> 21
   <212> DNA
   <213> Artificial Sequence
<220>
   <223> Oligonucleotide for amplifying the nucleotide sequence of Dermo-1 gene
<400> 14
   ggcaatggca gcatcattca g 21
<210> 15
   <211> 20
   <212> DNA
   <213> Artificial Sequence
<220>
   <223> Oligonucleotide for amplifying the nucleotide sequence of Sox9 gene
<400> 15
   gtcagccagg tgctcaaagg 20
<210> 16
   <211> 20
   <212> DNA
   <213> Artificial Sequence
<220>
   <223> Oligonucleotide for amplifying the nucleotide sequence of Sox9 gene
<400> 16
   acttgtaatc cgggtggtcc 20
<210> 17
   <211> 21
   <212> DNA
   <213> Artificial Sequence
<220>
   <223> Oligonucleotide for amplifying the nucleotide sequence of BMP-4 gene
<400> 17
   ttctgcagat gtttgggctg c 21
<210> 18
   <211> 20
   <212> DNA
   <213> Artificial Sequence
<220>
   <223> Oligonucleotide for amplifying the nucleotide sequence of BMP-4 gene
<400> 18
   agagccgaag ctctgcagag 20
<210> 19
   <211> 20
   <212> DNA
   <213> Artificial Sequence
<220>
   <223> Oligonucleotide for amplifying the nucleotide sequence of Wnt-5a gene
<400> 19
   ggatggctgg aagtgcaatg 20
<210> 20
   <211> 20
   <212> DNA
   <213> Artificial Sequence
<220>
   <223> Oligonucleotide for amplifying the nucleotide sequence of Wnt-5a gene
<400> 20
   acacaaactg gtccacgatc 20
<210> 21
   <211> 20
   <212> DNA
   <213> Artificial Sequence
<220>
   <223> Oligonucleotide for amplifying the nucleotide sequence of Versican gene
<400> 21
   acgatgccta ctttgccacc 20
<210> 22
   <211> 21
   <212> DNA
   <213> Artificial Sequence
<220>
   <223> Oligonucleotide for amplifying the nucleotide sequence of Versican gene
<400> 22
   tagtgaaaca caaccccatc c 21
<210> 23
   <211> 20
   <212> DNA
   <213> Artificial Sequence
<220>
   <223> Oligonucleotide for amplifying the nucleotide sequence of CD133 gene
<400> 23
   atggccctcg tactcggctc 20
<210> 24
   <211> 20
   <212> DNA
   <213> Artificial Sequence
<220>
   <223> Oligonucleotide for amplifying the nucleotide sequence of CD133 gene
<400> 24
   cacgcggctg taccacatag 20